# EUROPEAN PATENT APPLICATION

(11) **EP 4 607 143 A1**
(43) Date of publication of application: **27.08.2025**
(21) Application number: 24159626.1
(22) Date of filing: 26.02.2024
(51) Int. Cl.: G01B 9/02003, A61B 3/10, A61B 5/00, G01B 9/02004

(54) **OPTICAL COHERENCE TOMOGRAPHY INSTRUMENT AND OPTICAL COHERENCE TOMOGRAPHY METHOD**

(71) Applicant: Optos plc, Dunfermline, Scotland KY11 8GR (GB)
(72) Inventor: PRECIADO, Miguel, Dunfermline, Scotland, KY11 8GR (GB); NOVAK, Pavel, Dunfermline, Scotland, KY11 8GR (GB); URBAN, Dorian, Dunfermline, Scotland, KY11 8GR (GB)
(74) Representative: Hoffmann Eitle

(57) **Abstract**

An OCT instrument comprising: an optical coupler which generates signal light and reference light from a swept narrowband light source; a detector which samples a time-varying interference signal based on the reference light and signal light returned from a sample; an adjustable optical frequency shifter which: generates a first sideband light by adjusting an optical frequency of the reference light, such that the detector unit samples a first time-varying interference signal resulting from an interference between the sideband light and the returned signal light; and maintains the optical frequency of the reference light such that the detector unit samples a second time-varying interference signal resulting from an interference between the reference light and the returned signal light. The OCT instrument generates a respective axial depth profile of the sample based on each of the sampled first time-varying interference signal and the sampled second time-varying interference signal.

## Description

### [Field]

Example aspects herein relate to optical coherence tomography (OCT) and, more particularly, to OCT instruments and methods for performing an OCT measurement of a sample.

### [Background]

Optical coherence tomography (OCT) is an imaging technique capable of obtaining high-resolution measurements and imaging of surface and subsurface structures of, by example and without limitation, human tissue, particularly the retina, non-invasively.

In optical coherence tomography, measurement light is split into two paths by an optical coupler. The optical coupler directs light in each path to a different arm of an interferometer. One arm is termed a reference arm, while the other is termed a sample arm. In the sample arm, the light is directed by a sample optical system, sometimes termed a front-end optical system, to a sample under investigation (an imaging target), and reflected light is collected by the sample optical system and returned to the optical coupler. In the reference arm, the light enters a reference optical system which returns the light to the optical coupler. The returning light from the sample arm and the reference arm are recombined by the coupler to generate an interference pattern. The interference pattern is recorded by a detector.

The interference pattern contains information about the optical path travelled by the reflected signal sample and the magnitude of the signal sample having travelled a particular optical path length. Since the wavelength of the light is selected to at least partially penetrate the sample under investigation, the interference pattern contains information about surface and subsurface structures of the sample.

Different implementations of the optical coherence tomography technique are known in the art. One technique, termed swept-source optical coherence tomography (SS-OCT), uses measurement light, the optical frequency of which is periodically modulated in a controlled way across a defined source bandwidth. Typically, a series of rising sweeps over a defined optical frequency band are used as the modulation. A temporally varying interference pattern signal is recorded by the detector. A Fourier transform of the recorded signal over one periodic modulation of the optical frequency of the measurement light generates an axial depth profile of the sample, with intensity corresponding to a strength of the reflection.

Scanning the signal sample one- or two-dimensionally across the surface of the sample enables an axial depth profile to be obtained for each of a plurality of points across the surface of the sample, such that a two- dimensional depth profile (i.e. a B-scan) or a three-dimensional depth profile (i.e. a C-scan) of the sample can be obtained.

The coherence length of the measurement light determines the maximum imaging depth of the system (i.e. the depth of the depth profile), while the optical path length of the reference arm determines an axial position at which the axial depth profile is obtained. The coherence length of the measurement light can be high, such that the interference between the returning reflected signal sample and the returning reference light contains a wealth of information about the reflectivity of the sample over a large axial depth range. However, since some of the frequencies in the interference signal will be too high to be recorded by the detector, only information about a sub-range of this axial depth range, corresponding to the detection bandwidth of the detector, will be available from the Fourier transform of the recorded signal.

It would be useful to provide an optical coherence tomography instrument whose axial imaging depth range is not limited by the detection bandwidth in this way, and which is consequently capable of imaging the sample over a broader range of depths.

### [Summary]

There is provided, in accordance with a first example aspect herein, an OCT instrument comprising: an optical coupler arranged to accept light from a swept narrowband light source and to split the light into at least signal light and reference light; a reference optical system arranged to return the reference light; a front-end optical system arranged to direct the signal light towards a sample and to return signal light reflected from the sample; a detector unit arranged to sample at least one time-varying interference signal based on the returned reference light and the returned signal light; an adjustable optical frequency shifter; and a frequency shift controller. The frequency shift controller is arranged to control the adjustable optical frequency shifter to: generate a sideband light by adjustably increasing, adjustably decreasing, or adjustably both increasing and decreasing, an optical frequency of a part of one of the returned reference light or the returned signal light, such that the detector unit samples a first time-varying interference signal resulting from an interference between the sideband light and the other of the returned reference light or the returned signal light; and maintain the optical frequency of the one of the returned reference light or the returned signal light, such that the detector unit samples a second time-varying interference signal resulting from an interference between the returned reference light and the returned signal light. The OCT instrument further comprises a data processing unit, which is arranged to generate: a first axial depth profile of the sample based on the sampled first time-varying interference signal; and a second axial depth profile of the sample based on the sampled second time-varying interference signal.

In an example embodiment, the frequency shift controller may be operable in a first mode to control the adjustable optical frequency shifter to generate the sideband light, and in a second mode to control the adjustable optical frequency shifter to maintain the optical frequency of the one of the returned reference light or the returned signal light, and the front-end optical system may be arranged to scan the signal light across the sample in a first direction along a scan path on the sample during operation of the frequency shift controller in the first mode, and along the scan path in a second direction opposite to the first direction during operation of the frequency shift controller in the second mode.

There is provided, in accordance with a second example aspect herein, an OCT instrument comprising: an optical coupler arranged to accept light from a swept narrowband light source and to split the light into at least signal light and reference light; a reference optical system arranged to return the reference light; a front-end optical system arranged to direct the signal light towards a sample and to return signal light reflected from the sample; a detector unit arranged to sample at least one time-varying interference signal based on the returned reference light and the returned signal light; an adjustable optical frequency shifter; and a frequency shift controller. The frequency shift controller is arranged to control the adjustable optical frequency shifter to: generate a first sideband light by adjustably increasing, adjustably decreasing, or adjustably both increasing and decreasing an optical frequency of a part of one of the returned reference light or the returned signal light, such that the detector unit samples a first time-varying interference signal resulting from an interference between the first sideband light and the other of the returned reference light or the returned signal light; and generate a second sideband light of different optical frequency than the first sideband light, by adjustably increasing or decreasing an optical frequency of a part of the one of the returned reference light or the returned signal light, such that the detector unit samples a second time-varying interference signal resulting from an interference between the second sideband light and the other of the returned reference light or the returned signal light. The OCT instrument further comprises a data processing unit, which is arranged to generate: a first axial depth profile of the sample based on the sampled first time-varying interference signal; and a second axial depth profile of the sample based on the sampled second time-varying interference signal.

In an example embodiment of the OCT instrument of the second example aspect, the frequency shift controller may be operable in a first mode to control the adjustable optical frequency shifter to generate the first sideband light, and in a second mode to control the adjustable optical frequency shifter to generate the second sideband light, and the front-end optical system may be arranged to scan the signal light across the sample in a first direction along a scan path on the sample during operation of the frequency shift controller in the first mode, and along the scan path in a second direction opposite to the first direction during operation of the frequency shift controller in the second mode.

In the OCT instrument of the second example aspect of the example embodiment thereof, the reference optical system may be non-electromechanical, thus having no electrically driven moving parts for varying an optical path length of the reference light.

In any of the foregoing, the sample may comprise an eye of a subject, and the first axial depth profile may comprise an axial depth profile of a portion of a retina of the eye, and the second axial depth profile may comprise an axial depth profile of a portion of an anterior segment of the eye.

Alternatively, in a further example embodiment, the OCT instrument may be arranged to acquire, as the first axial depth profile of the sample, a B-scan representing a section of the sample, the sample in the section being inclined with respect to a plane normal to an axial direction along which depth information of the B-scan is acquired, the front-end optical system may be arranged to scan the signal light across a plurality of scan locations along the section of the sample, the detector unit may be arranged to sample, for each of the scan locations, a respective time-varying interference signal resulting from an interference between the (first) sideband light and the other of the returned reference light or the returned signal light, the data processing unit may be arranged to generate the B-scan based on the sampled time-varying interference signals, and the frequency shift controller may be further arranged to control the adjustable optical frequency shifter to vary the optical frequency during the scan to compensate for the inclination of the sample in the section, such that an image (or representation) of the sample in the B-scan has less of an inclination relative to a lateral direction in the B-scan than would be present in a B-scan of the section of the sample acquired by the optical coherence tomography instrument without the control by the frequency shift controller. In this case, the frequency shift controller may be arranged to control the adjustable optical frequency shifter to vary the optical frequency during the acquisition of the B-scan to compensate for the inclination of the sample in the section using one of: a pre-stored estimate of the inclination of the sample in the section; or pre-stored calibration data indicative of a measured inclination of the sample in the section. The pre-stored calibration data may be determined from a calibration B-scan acquired by the OCT instrument without varying the optical frequency during the scan, for example. In either case, the sample may comprise a retina of an eye of a subject.

In any of the foregoing, the reference optical system may comprise one of a reflector arranged to reflect the reference light to return the reference light, or an optical loop to return the reference light, the optical loop optionally having a fixed optical path length. Additionally or alternatively, one of the reference light or the signal light may pass by way of the adjustable optical frequency shifter in forward and reverse directions. Additionally or alternatively, the optical frequency shifter may include an acousto-optic modulator or an electro-optic modulator.

In any of the foregoing, the OCT instrument may further comprise the swept narrowband light source, wherein the swept narrowband light source is arranged to emit the light to the optical coupler, and the light is narrowband light. The swept narrowband light source may be configured to periodically vary an optical frequency of the light emitted thereby.

There is provided, in accordance with a third example aspect herein, a method of generating OCT axial depth profiles of a sample. The method comprises: splitting light from a swept narrowband light source into at least signal light propagating to the sample along a sample arm of an interferometer and reference light propagating along a reference arm of the interferometer; generating a sideband light by adjustably increasing or decreasing an optical frequency of a part of one of (i) reference light which has been returned by the reference arm, or (ii) signal light which has been returned from the sample via the sample arm; sampling a first time-varying interference signal resulting from an interference between the sideband light and the other of the returned reference light or the returned signal light; sampling a second time-varying interference signal resulting from an interference between the returned reference light and the returned signal light; and generating a first OCT axial depth profile of the sample based on the sampled first time-varying interference signal, and a second OCT axial depth profile of the sample based on the sampled second time-varying interference signal.

There is provided, in accordance with a fourth example aspect herein, a method of generating OCT axial depth profiles of a sample. The method comprises: splitting light from a swept narrowband light source into at least signal light propagating to the sample along a sample arm of an interferometer and reference light propagating along a reference arm of the interferometer; generating a first sideband light by adjustably increasing or decreasing an optical frequency of a part of one of (i) reference light which has been returned by the reference arm, or (ii) signal light which has been returned from the sample via the sample arm; generating a second sideband light of different optical frequency than the first sideband light, by adjustably increasing or decreasing an optical frequency of a part of the one of the returned reference light or the returned signal light; sampling a first time-varying interference signal resulting from an interference between the first sideband light and the other of the returned reference light or the returned signal light; sampling a second time-varying interference signal resulting from an interference between the second sideband light and the other of the returned reference light or the returned signal light; and generating a first OCT axial depth profile of the sample based on the sampled first time-varying interference signal, and a second OCT axial depth profile of the sample based on the sampled second time-varying interference signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an OCT instrument according to a first example embodiment herein.
Figure 2 is a graphical representation of a periodic frequency sweep provided by a swept source typical of OCT instruments.
Figure 3 shows part of a conventional OCT instrument including a reference arm and a sample arm.
Figure 4 shows an effect of introducing an optical delay to the periodic frequency sweep of Figure 2 by means of an increased optical path length.
Figure 5 is a schematic illustration showing parts of the OCT instrument according to the first example embodiment herein, including reference and sample arms.
Figure 6 shows an effect of introducing an optical frequency downshift to the periodic frequency sweep of Figure 2 by means of an optical frequency shifter.
Figure 7 shows an effect of introducing a reverse optical delay to the periodic frequency sweep of Figure 2 by means of an increased optical path length.
Figure 8 shows an effect of introducing an optical frequency upshift to the periodic frequency sweep of Figure 2 by means of an optical frequency shifter.
Figure 9 is a schematic illustration showing a variant of the OCT instrument of Figure 5, in which an adjustable optical frequency shifter 70 is arranged between an optical coupler 10 and a reference optical system 30.
Figure 10 is a schematic illustration of an OCT instrument according to an example embodiment herein, in which an adjustable optical frequency shifter 70 is in a front-end optical system.
Figure 11 is a schematic illustration of a variant of the OCT instrument of Figure 10, in which an adjustable optical frequency shifter 70 is arranged between an optical coupler 10 and a front-end optical system 40.
Figure 12 shows an example of a set of sidebands generated during a frequency sweep.
Figure 13(a) shows an example conventional B-scan, wherein a real cross-sectional image R of a part of a plastic cylinder is overlapped by a complex conjugate mirror artifact A.
Figure 13(b) shows an example B-scan acquired using techniques described herein, wherein a real cross-sectional image R of a part of a plastic cylinder is not overlapped by a complex conjugate mirror artifact A.
Figure 14(a) is a schematic illustration of a calibration B-scan of a sample.
Figure 14(b) is a schematic illustration of a varying control signal, which can be used by the frequency shift controller 80-1 to drive the adjustable optical frequency shifter 70 during the acquisition of a B-scan in the first example embodiment.
Figure 14(c) is a schematic illustration of a flattened B-scan acquired using the control signal of Figure 14(b).
Figure 15 is a schematic illustration of an OCT instrument according to a second example embodiment herein.
Figure 16 is a schematic illustration showing parts of an OCT instrument according to another example embodiment herein.
Figure 17 is a schematic illustration showing parts of an OCT instrument according to yet another example embodiment herein.
Figure 18 is a schematic illustration of an OCT instrument using balanced detection, according to still a further example embodiment herein.
Figure 19 is a schematic illustration of an OCT instrument using balanced detection, according to another example embodiment herein.
Figure 20 is a schematic illustration of an integrated controller for controlling an OCT instrument according to an example embodiment herein.
Figure 21 is a flow diagram illustrating a method of generating OCT axial depth profiles of a sample in the first example embodiment.
Figure 22 is a flow diagram illustrating a method of generating OCT axial depth profiles of a sample in the second example embodiment.

### [Detailed Description of Example Embodiments]

### [First Example Embodiment]

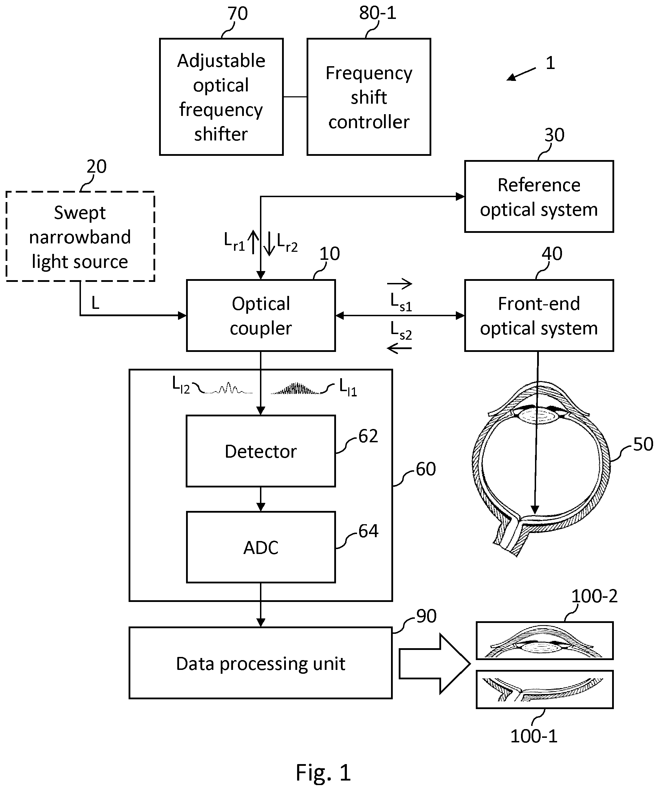

Figure 1 is a schematic diagram of an OCT instrument (also referred to herein as an OCT system) 1 according to a first example embodiment herein.

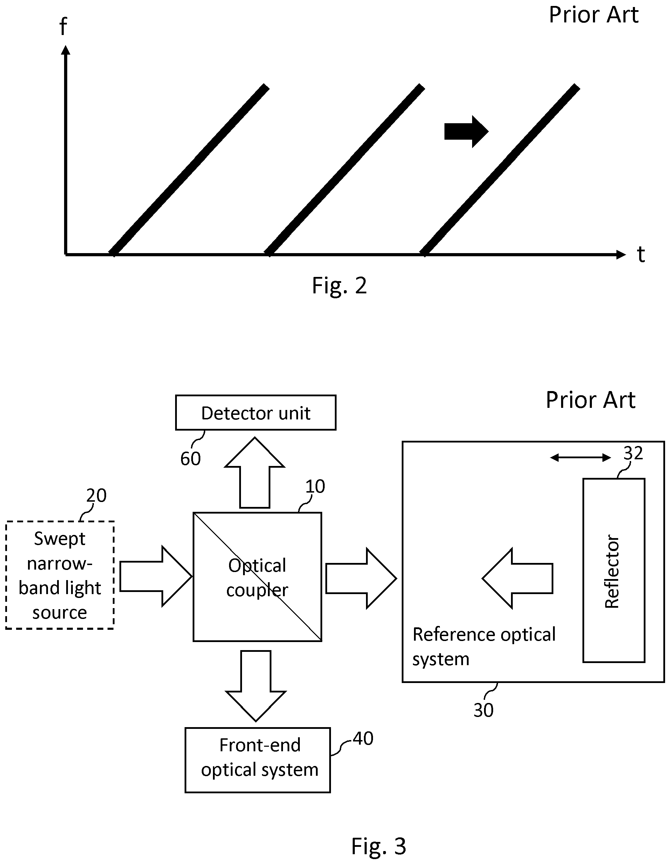

In the (swept-source) OCT instrument 1, an optical coupler 10 receives a beam of narrowband light L with a varying centre frequency, which is generated by a swept narrowband light source 20. The swept narrowband light source 20 is configured to vary the centre frequency of the narrowband light L in a repetitive manner, such as by a repeated periodic frequency sweep over a defined frequency band between a lower frequency and an upper frequency. Such a periodic frequency sweep is exemplified in Figure 2, which shows the frequency of an output of such swept narrowband light source 20 with respect to time. Such a frequency sweep is conventionally termed a chirp. The sweep may rise in time from low frequency to high frequency, or may fall in time from high frequency to low frequency. The sweep may also successively rise and fall in time. For example, a forward sweep from low to high frequency may be followed by a reverse sweep from high to low frequency. The sweep range may be of the order of, for example, 100 nm. The swept narrowband light source 20 may have high coherence, and for example may have a coherence length in excess of 5 mm. In some configurations, the coherence length may exceed 10 mm, or may even exceed 100 mm. Of course, these examples are non-limiting.

The swept narrowband light source 20 may be a (wavelength-) swept laser or a swept laser diode, for example an external cavity laser, an optical parametric amplifier, a Fourier-domain mode-locking laser (FDML) or a swept vertical cavity surface-emitting laser (VCSEL), although these examples are not limiting to the scope of the invention. The bandwidth of the swept narrowband light source 20 may be selected for optimum penetration through, for example, a lens of an eye under examination and tissues of a retina of the eye. Also, the bandwidth may be, as in the present example embodiment, be in the infra-red region of the optical spectrum, for example, at wavelengths longer than 850 nm. Example wavelengths which may be included in the sweep are 850 nm, 1050 nm, 1310 nm or 1550 nm, although these examples are not limiting.

The beam of narrowband light L generated by the swept narrowband light source 20 is directed to the optical coupler 10, which may, as in the present example embodiment, be provided in the form of a beam splitter. The beam splitter splits the beam of narrowband light L into two beams. One of the beams propagates to a reference arm of an interferometer of the OCT instrument 1, which comprises a reference optical system 30. Meanwhile, the other beam propagates to a sample arm of the interferometer, which comprises a front-end optical system 40 (which may also be referred to as a sample optical system). The splitting of the beam L may be symmetric, such that equal intensity is directed into the resulting two beams, or may be asymmetric, such that unequal intensity is directed into the resulting two beams.

A first beam, also referred to herein as a signal light (or sample light) Lₛ₁, is directed to the front-end optical system 40, which includes optical components to shape and direct the light beam to a sample 50 such as, by example only, an eye of a subject, and to collect reflected signal light Lₛ₂ (i.e. signal light reflected from the sample 50) and return it to the optical coupler 10. The reflected signal light Lₛ₂ follows essentially the same path to the optical coupler 10 as the signal light Lₛ₁, but in a reverse direction.

The front-end optical system 40 may, as in the present example embodiment, include conventional components known in the field of optical coherence tomography, and such conventional components may be adapted according to the imaging operation to be performed. More particularly, the front-end optical system 40 may, as in the present example embodiment, include scanning optics which can cause a pivoting of the signal light Lₛ₁ about a pivot point located in an anterior segment of the eye in order to scan the signal light Lₛ₁ across a wide field of the retina located in a posterior segment of the eye.

The front-end optical system 40 may, as in the present example embodiment, include one or more scanners arranged to scan the beam of signal light Lₛ₁ in one or more directions across the sample 50 (e.g. the retina of a subject eye). Such scanners can include oscillating plane mirrors such as galvanometer scanners, MEMS mirrors, rotating mirrors, prism or polygon scanners or resonant scanners.

The front-end optical system 40 may also include a scan relay unit comprising, for example, a lens or curved mirror, arranged to image one scanner which scans the beam of signal light Lₛ₁ in a first direction onto a subsequent second scanner, which scans the beam in a second, different direction, thereby to generate a two-dimensional scan pattern arising from an apparent origin located at the second scanner.

The front-end optical system 40 may also include a scan transfer unit, for example one or more lenses or a curved mirror, arranged to project the apparent origin into space beyond the front-end optical system 40. In this way, the two-dimensional scan pattern arising from the apparent origin at the second scanner is transferred to an apparent pivot point in the space beyond the front-end optical system 40, such that the beam of signal light Lₛ₁ pivots about the pivot point in the scan.

Referring again to the optical coupler 10 in Figure 1, a second beam, also referred to herein as a reference light Lᵣ₁, is directed by the optical coupler 10 to a reference optical system 30, which is configured to return the reference light to the optical coupler 10. At the optical coupler 10, the returning reflected signal sample Lₛ₂ (i.e. the signal light reflected from the sample 50) and the returning reference light Lᵣ₂ (i.e. the reference light returned from the reference optical system 30) are combined to interfere with one another, and are directed as interfering beams to detector unit 60. The detector unit 60 may, as in the present example embodiment, comprise a detector 62 and an analogue-to-digital converter (ADC) 64. The detector 62 may comprise a photodetector such as a photodiode or an avalanche photodiode, which converts an optical intensity of the interfering beams to provide a resulting converted signal in the form of, e.g. an electrical signal such as a voltage or current. The resulting converted signal may, as in the present example embodiment, be a time-varying analogue signal. After being output by the detector 62, the resulting converted signal may be recorded in a time-varying manner, as will be described below, wherein a recorded version of the signal constitutes an interferogram between the returning reflected signal light Lₛ₂ and the returning reference light Lᵣ₂ with respect to the optical frequency of the narrowband light from the swept narrowband light source 20.

In the first example embodiment depicted in Figure 1, the ADC 64 periodically samples and quantises the signal output by the detector 62 with a predetermined sample frequency, and then digital values of the quantised and sampled signal are provided to a data processing unit 90, wherein the values are recorded. Accordingly, the detector 62 and the ADC 64 together constitute the detector unit 60, which samples a time-varying interference signal between the returning reference light Lᵣ₂ and the returning signal light Lₛ₂. The data processing unit 90 may, as in the present example embodiment, perform a Fourier transform operation, such as a fast Fourier transform, on the time-varying quantised and sampled signal values obtained from the ADC 64 to generate an axial depth profile, which may be provided in the form of a one-, two- or three-dimensional data array (depending on whether the measurement beam is scanned across the sample 50 and, if so, the dimensionality of the scan).

As a result of the periodic sampling of the output of detector 62 by the ADC 64, the highest-frequency components of the time-varying analogue signal, which lie outside a detection bandwidth of the system, will be aliased, potentially compromising the image. The bandwidth of the system is defined as a combination of electronic bandwidth, which determines the highest frequency that can be detected, and the bandwidth of the ADC 64, which determines the range of frequencies which can be recorded uniquely (i.e. without aliasing). An anti-aliasing filter, which matches the bandwidth of the ADC 64, may be provided between the detector 62 and the ADC 64.

The light source 20 may have relatively high coherence length such that the interference between the returning reflected signal light Lₛ₂ and the returning reference light Lᵣ₂ contains information about the reflectivity of the sample 50 over a large axial depth range defined by the high coherence length. Nonetheless, only information about a sub-range of this axial depth range corresponding to the detection bandwidth, centred on an axial position defined by the optical path length of the reference arm, is available from a Fourier transform of the sampled signal recorded at the data processing unit 90.

In conventional OCT instruments, the optical path length of the reference arm is adjusted to set an axial position of the measured axial depth range to correspond to the position of the surface of the sample under investigation, in view of the typically low coherence length of the light source used. Thereby, an optical delay is introduced between the signal sample and the reference light, which ensures that the signal sample and the reference light maintain coherence when combined to interfere at the detector.

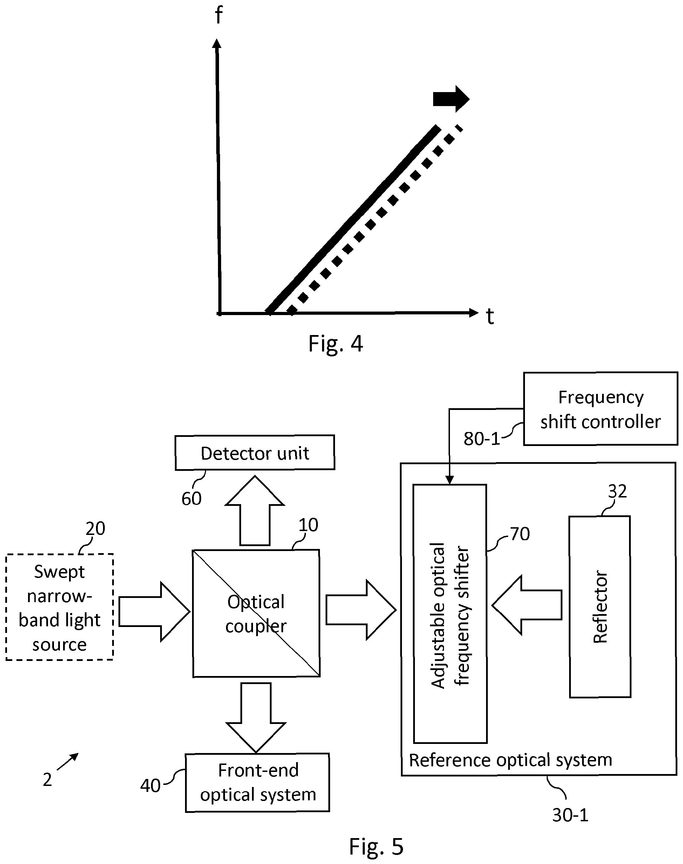

An exemplary configuration of such an arrangement is shown in Figure 3, in which a moveable reflector 32, such as a movable mirror, is provided in the reference optical system 30 to alter the optical path length travelled by light in the reference arm. Such an alteration to the optical path length travelled by light in the reference arm corresponds to a variable optical delay of the frequency chirp generated by the swept light source 20, as shown in Figure 4. In the present configuration, an optical delay is introduced to the reference light Lᵣ₂. It should be understood that the optical delay is relative to the signal sample Lₛ₂ and may, in principle, be positive or negative, depending on whether the optical path of the sample arm is greater or smaller in length than the optical path of the reference arm.

The present inventors have recognised that when the swept narrowband light source 20 has sufficiently high coherence, a constant frequency shift, such as a frequency upshift or downshift, applied to the frequency chirp generated by the swept narrowband light source 20, corresponds substantially to a constant optical delay, with the amount of equivalent delay introduced corresponding to the amount of frequency shift introduced.

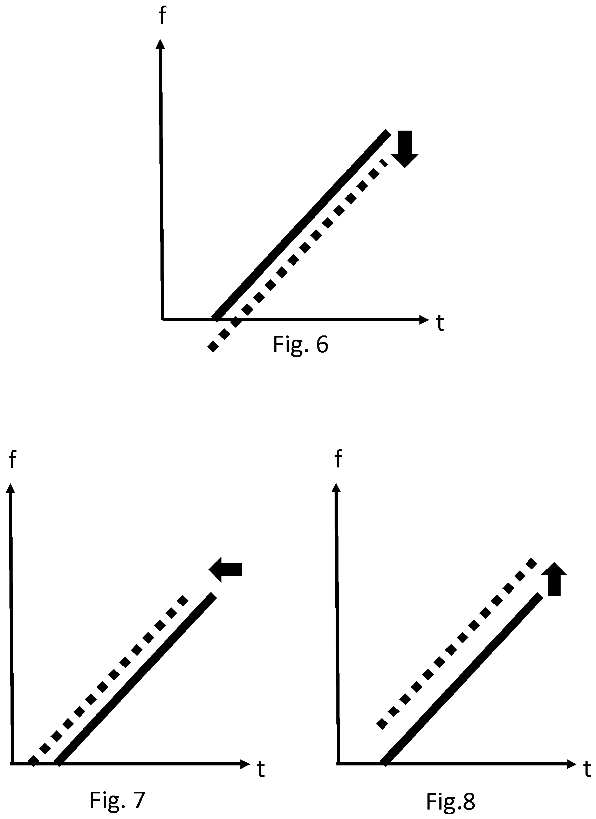

Such a frequency shift may be introduced by placing an adjustable optical frequency shifter 70 into the reference arm, for example in a reference optical system 30-1 (as an example of reference optical system 30), between the optical coupler 10 and the moveable reflector 32, as shown in OCT instrument 2 of Figure 5, which is an example implementation of OCT instrument 1. The effect on the frequency chirp generated by the swept narrowband light source 20 is shown in Figure 6, from which it can be seen, by comparison with Figure 4, that the introduction of a predetermined frequency shift is equivalent to the introduction of a predetermined optical delay. Whereas Figures 4 and 6 show the effect of introducing a frequency downshift, a frequency upshift is also contemplated. Figures 7 and 8 correspond to Figures 4 and 6 for the case of a frequency upshift.

Referring to Figure 5, the reference light can travel twice through adjustable optical frequency shifter 70, in particular, in one instance in the forward direction from optical coupler 10 to reflector 32 by way of the adjustable optical frequency shifter 70, and in another instance in the reverse direction from reflector 32 to optical coupler 10 by way of the adjustable optical frequency shifter 70. The predetermined modulation is introduced on each pass through the adjustable optical frequency shifter 70. Hence, in the example configuration of Figure 5, the applied modulation is effectively applied twice. This would allow for the amplitude of the modulating signal to be reduced to achieve the same effect in the case of phase modulation, or could excite higher order harmonics in the case of amplitude modulation or phase modulation.

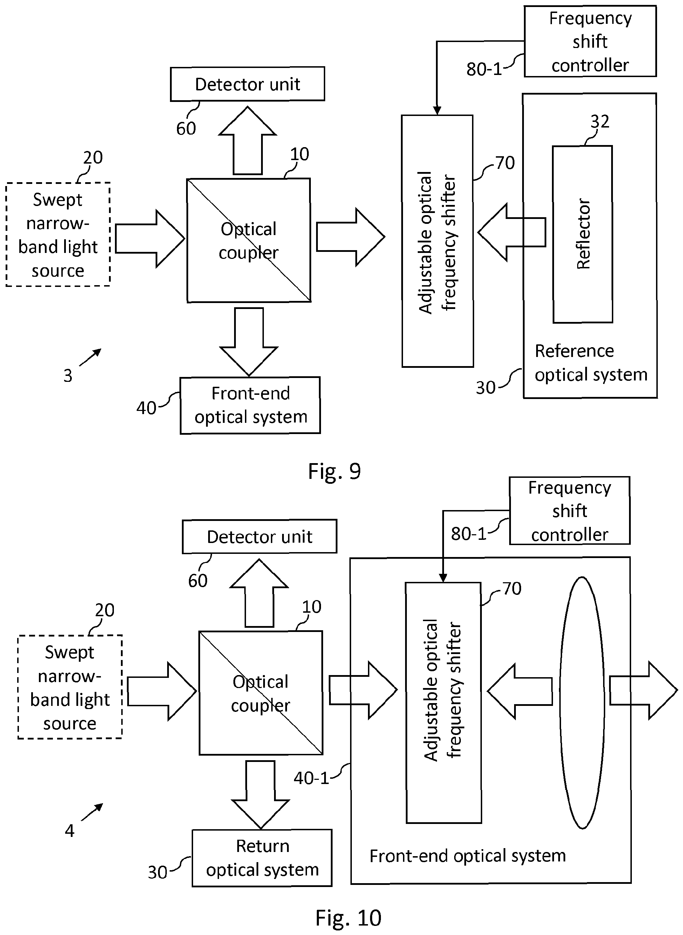

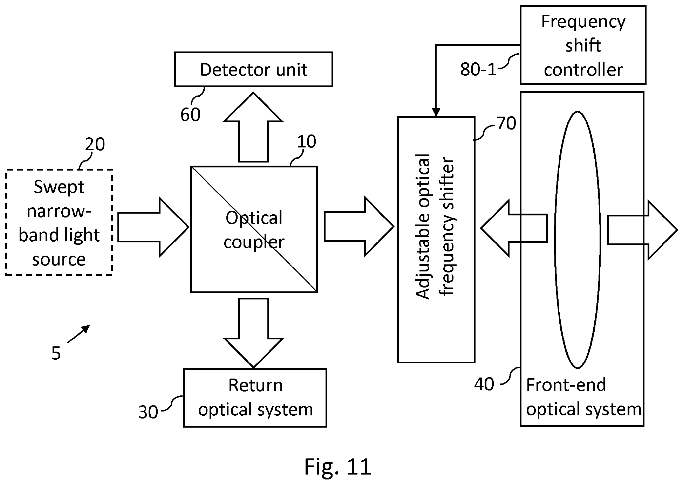

Equivalently, the adjustable optical frequency shifter 70 may be arranged between the optical coupler 10 and the reference optical system 30, as shown in Figure 9. Similarly, by placing the adjustable optical frequency shifter 70 into a front-end optical system 40-1 (as an example of front-end optical system 40), as shown in the OCT instrument 4 of Figure 10, a frequency shift may be introduced to the sample arm which would correspond to an optical delay in the sample arm. Equivalently, the adjustable optical frequency shifter 70 may be arranged between the optical coupler 10 and the front-end optical system 40, as shown in the OCT instrument 5 of Figure 11. The adjustable optical frequency shifter 70 may thus be placed in one of various optical paths in the OCT instrument 1, so as to adjustably increase or decrease an optical frequency of a part of the returned reference light Lᵣ₂ or the returned signal light Lₛ₂ (as the case may be).

As illustrated in Figure 1, the adjustable optical frequency shifter 70 is controllable by a frequency shift controller 80-1. By virtue of the frequency shift controller 80-1 controlling the adjustable optical frequency shifter 70 to adjust the amount of frequency shift introduced, an effect equivalent to introducing an adjustable optical delay by means of, for example, a movable reflector 32 as shown in Figure 3, can be achieved, without using any moving parts. The reference optical system 30 may thus be a non-electromechanical (or solid-state) reference optical system, having no electrically driven moving parts to vary the optical path length of the reference arm. Furthermore, by virtue of the frequency shift controller 80-1 being operable in at least a first mode and a second mode as described in more detail below, the OCT instrument 1 may be able to rapidly acquire a first axial depth profile (e.g. a first B-scan) and a second axial depth profile (e.g. a second B-scan) of the sample 50, wherein the axial depth profiles cover (image) different (overlapping or non-overlapping) axial depth ranges of the sample 50. With the optoelectronic reference arm of the OCT instrument 1 having no moving parts to change the optical path length of the reference beam, the acquisition of the axial depth profiles can occur on a timescale on which relative movement of the sample 50 with respect to the OCT instrument 1 is negligible. Where the axial depth profiles are B-scans, the B-scans may be combined to produce a composite B-scan wherein the relative orientations and positional relationships of anatomical features imaged in the component B-scans are preserved. The OCT instrument 1 may thus be able to acquire useful anatomical information over a broad axial depth range that exceeds the limit imposed by the available detector bandwidth where conventional imaging techniques are used.

By introducing a predetermined amount of frequency upshift or downshift to the light in the reference arm (or sample arm, as the case may be), the temporal interference frequency between the returning reflected light Lₛ₂ of the sample beam and the returning light Lᵣ₂ of the reference beam can be slowed so that interference components arising from a desired axial region of the sample 50 lie within the available system bandwidth defined by the photodetector or a low-pass filter provided after the photodetector, where the frequency cut-off of the low-pass filter is matched to the Nyquist frequency of the ADC 64. Stated simply, the beat frequency of the interferogram components arising from the desired axial region of the sample 50 is slowed sufficiently to enable it to be recorded. Consequently, the electrical frequency of the electrical signal output by the detector 62 is also reduced. As one example, the frequency of the interferogram components arising from the desired axial region of the sample 50 may be reduced to lower than the Nyquist frequency of the ADC 64.

The adjustable optical frequency shifter 70 may be provided in one of many different forms. As one example, the adjustable optical frequency shifter 70 may be provided in the form of an acousto-optic modulator (AOM).

An acousto-optic modulator comprises a driven optical medium to which vibrations are applied at a defined frequency. The vibrations induce phonons in the optical medium which interact with photons passing through the optical medium to generate diffracted light having a frequency shift proportional to the frequency of the vibration, with the constant of proportionality determined by the order of diffraction. Accordingly, by varying the frequency of the vibration, a well-defined optical frequency shift may be introduced. Depending on the order of diffraction, and particularly whether the diffractive order is a positive or negative order, the optical frequency shift is either an upshift or downshift. Such acousto-optic modulators can operate in a travelling-wave configuration or a standing wave configuration. In such a configuration, the frequency shift controller 80-1 is a radio frequency driver arranged to apply a variable radio frequency electric signal to the optical medium using a piezo-electric element attached to the optical medium.

Alternatively, the adjustable optical frequency shifter 70 may, as in the present example embodiment, be provided in the form of an electro-optic modulator (EOM), which can similarly be used to introduce the optical frequency shift.

An electro-optic modulator comprises an optical medium exhibiting an electro-optical effect to which an electric field is applied. The electric field may be applied to the medium, for example, by placing the medium between the plates of a capacitor. The applied electric field induces a corresponding change in the refractive index of the optical medium according to the strength of the electric field, due to the electro-optic effect. Without wishing to be bound by theory, such change in refractive index may typically be caused by forces that distort the position, orientation and/or shape of the molecules constituting the optical medium. The change in refractive index induces a change in the phase of the light exiting the medium. If the electric field is varied with a defined frequency, the refractive index varies also with that defined frequency. The varying of the refractive index may induce a correspondingly varying change in the phase of the light exiting the optical medium.

For example, if the electric field is varied sinusoidally at a predetermined frequency ω, then a time-dependent phase at the predetermined frequency ω may be added to the time dependence of the electromagnetic wave of the light, for example of frequency Ω exiting the optical medium. As a result of the addition of this time-dependent phase, a set of sidebands comprising at least a first pair of sidebands (sideband lights) are added to the light, at frequency Ω ± ω, each sideband of the first pair of sidebands shifted in frequency by the predetermined frequency ω relative to the frequency Ω of the light.

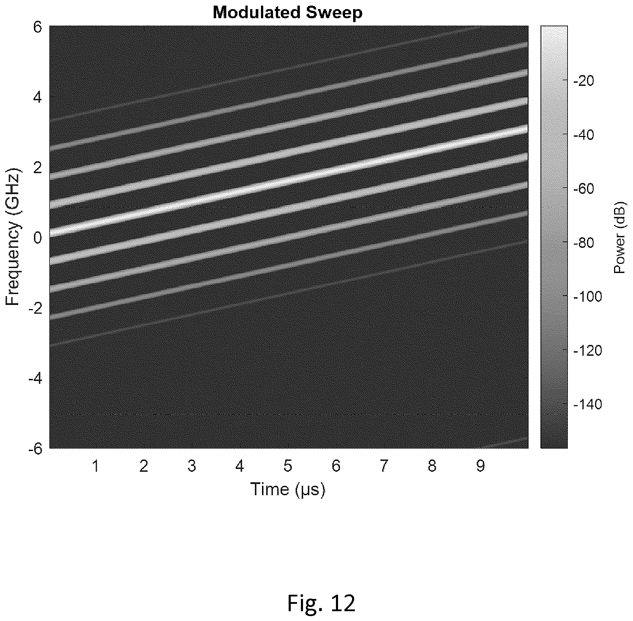

Accordingly, by varying the frequency of the electric field, a well-defined optical frequency shift may be introduced to the light. Due to the presence of two sidebands, an optical frequency shift which is an upshift or downshift can be obtained. An example of a set of sidebands generated during a frequency sweep is illustrated in Figure 12.

In another example embodiment, the electro-optic modulator (EOM) may alternatively be used to introduce the optical frequency shift by means of amplitude modulation, rather than phase modulation.

In particular, a phase-modulating electro-optic modulator can be used to introduce amplitude modulation to an incident light by incorporating at least one such electro-optic modulator into a corresponding at least one arm of an interferometer, such as a Mach-Zehnder interferometer, to which the incident light is applied. Such an interferometer has two arms, into which the incident light is coherently separated, and which generate output light by coherently combining the light of the two arms. The coherent separation and combining of the light can be performed by one or more beam-splitters. This arrangement is sometimes termed a Mach-Zehnder modulator (MZM) or an electro-optic amplitude modulator. In related arrangements, one electro-optic modulator can be provided to each of two arms of the interferometer.

Applying an electric field across the optical medium of the electro-optic modulator in the interferometer introduces a phase shift according to the strength of the electric field, as described above. Depending on the introduced phase shift, the amplitude of the light output from the interferometer will also vary according to the amount of phase shift introduced, in accordance with the normal principles of an interferometer. Thereby, a varying amplitude can be imparted to the light. The magnitude of the variation of the applied electric field may be set such that the difference in introduced phase shift between minimum and maximum applied electric field corresponds to half a wavelength of the input light. In such a configuration, a variation in the applied electric field at a predetermined frequency ω results in a variation in the amplitude of the output light also with a predetermined frequency ω.

For example, if the electric field is varied sinusoidally at a predetermined frequency ω, then a time-dependent amplitude at the predetermined frequency ω may be added to the time dependence of the electromagnetic wave of the light, for example of frequency Ω exiting the optical medium. As a result of the addition of this time-dependent amplitude, a set of sidebands comprising only a first pair of sidebands are added to the light at frequencies at frequency Ω ± ω, each sideband of the sidebands shifted in frequency by the predetermined frequency ω relative to the frequency Ω of the light. Accordingly, by varying the frequency of the electric field, a well-defined optical frequency shift may be introduced to the light. Due to the presence of two sidebands, an optical frequency shift which is an upshift or downshift can be obtained.

When sidebands are generated as frequency-shifted light, for example using an electro-optic phase modulator (as in the present example embodiment) or an electro-optic amplitude modulator, it may be necessary to select only the electrical signal corresponding to the sideband light for further use. Selecting the electrical signal corresponding to the upper sideband light can be performed by incorporating, for example, a suitable filter (e.g. a band-pass filter or a high-pass filter) between the detector 62 and the ADC 64, whereas selecting the electrical signal corresponding to the lower sideband light can be performed by incorporating, for example, a filter (e.g. a band-pass filter or a low-pass filter) between the detector 62 and the ADC 64 which is suitable for extracting the electrical signal corresponding to the lower sideband light. However, where the sidebands are sufficiently different in frequency from the incident light, then it may not be needed to include a filter. For example, by using an electro-optic phase modulator or an electro-optic amplitude modulator, a frequency offset of several gigahertz can be achieved for the shifted light compared with the input light. In such a situation, a filter may be omitted.

The frequency shift controller 80-1 may, as in the present example embodiment, be operable in a first mode to control the adjustable optical frequency shifter 70 to generate a first sideband light (e.g. from the first sideband, which typically has more optical power than higher sidebands) by adjustably increasing or decreasing the optical frequency of some of the returned reference light Lᵣ₂, such that the detector unit 60 samples a first time-varying interference signal L_{I1} resulting from an interference between the sideband light and the returned signal light Lₛ₂ as the optical frequency of the light L is being swept by the swept narrowband light source 20. In other example embodiments (where the adjustable frequency shifter is provided in the sample arm), the frequency shift controller 80-1 may be operable in the first mode to control the adjustable optical frequency shifter 70 to generate the first sideband light by adjustably increasing or decreasing the optical frequency of some of the returned signal light Lₛ₂, such that the detector unit 60 samples a first time-varying interference signal L_{I1} resulting from an interference between the sideband light and the returned reference light Lᵣ₂ as the optical frequency of the light L is being swept by the swept narrowband light source 20.

The front-end optical system 40 may, as in the present example embodiment, be arranged to scan the signal light Lₛ₁ across the sample 50 in a first direction along a scan path on the sample 50 during control of the adjustable optical frequency shifter 70 in the first mode, such that the OCT instrument 1 acquires a first axial depth profile 100-1 in the form of a first B-scan showing a section of the sample 50 along the scan path.

The centre of the imaging depth range of the OCT instrument 1 in the first mode may be rapidly adjusted in the axial direction of the OCT instrument 1, along which the sample beam propagates towards the sample 50, by controlling the adjustable optical frequency shifter 70 to apply an appropriate optical frequency shift. Where the sample 50 is an eye of a subject, the centre of the imaging depth range of the OCT instrument 1 in the first mode may as in the present example embodiment, be set to coincide with the retina of the eye such that the first axial depth profile 100-1 (B-scan) shows a section of the retina, as shown in Figure 1.

The frequency shift controller 80-1 may, as in the present example embodiment, be further operable in a second mode to maintain the optical frequency of the returned reference light Lᵣ₂, such that the detector unit 60 samples a second time-varying interference signal L_{I2} resulting from an interference between the returned reference light Lᵣ₂ and the returned signal light Lₛ₂ as the optical frequency of the light L is being swept by the swept narrowband light source 20. In the second mode, the frequency shift controller 80-1 may apply no drive signal to the adjustable optical frequency shifter 70, such that the adjustable optical frequency shifter 70 proves no frequency shift to the returned reference light Lᵣ₂. In other example embodiments (where the adjustable frequency shifter is provided in the sample arm), the frequency shift controller 80-1 may be operable in the second mode to maintain the optical frequency of the returned signal light Lₛ₂, such that the detector unit 60 again samples a second time-varying interference signal L_{I2} resulting from an interference between the returned reference light Lᵣ₂ and the returned signal light Lₛ₂ as the optical frequency of the light L is being swept by the swept narrowband light source 20.

During control of the adjustable optical frequency shifter 70 in the second mode, which follows its operation in the first mode, the front-end optical system 40 may, as in the present example embodiment, be arranged to scan the signal light Lₛ₁ across the sample 50 along the scan path mentioned above but in a second direction opposite to the first direction, such that the OCT instrument 1 acquires a second axial depth profile 100-2 in the form of a second B-scan showing a section of the sample 50 along the scan path.

The centre of the imaging depth range of the OCT instrument 1 in the second mode may be adjusted in the axial direction of the OCT instrument 1 by appropriately moving the OCT instrument 1 relative to the sample 50 (or vice versa), or by appropriate movement of a reflector in the reference arm of the OCT instrument in cases where the reference arm is electromechanical (or mechanical) and such movement is possible. Where the sample 50 is an eye of a subject, the centre of the imaging depth range of the OCT instrument 1 in the second mode may, as in the present example embodiment, be set to coincide with a point (e.g. a centre of the pupil) in the anterior segment of the eye, such that the second axial depth profile 100-2 (B-scan) shows a section of the anterior segment, as shown in Figure 1.

The OCT instrument 1 may thus be used to acquire a B-scan of the anterior segment of an eye and a B-scan of the retina of the eye through a common section of the eye, in rapid succession, by operation of the frequency shift controller 80-1 in the second mode while the OCT instrument 1 performs a forward scan to acquire an axial depth profile 100-2 in the form of a B-scan of the anterior segment of the eye, and by subsequent operation of the frequency shift controller 80-1 in the first mode while the OCT instrument 1 performs a reverse (back) scan along the same scan path to acquire a further axial depth profile 100-1, in the form of a B-scan of the retina of the eye. The order in which the anterior segment and retina of the eye are imaged may be reversed, such that the frequency shift controller 80-1 first operates in the first mode while the OCT instrument 1 performs a forward scan to acquire a B-scan of the retina of the eye, and by subsequent operation of the frequency shift controller 80-1 in the second mode while the OCT instrument 1 performs a reverse (back) scan along the same scan path to acquire a B-scan of the anterior segment of the eye. Furthermore, the anterior segment of the eye may alternatively be imaged during operation of the frequency shift controller 80-1 in the first mode, and the retina of the eye may be imaged during operation of the frequency shift controller 80-1 in the second mode.

The data processing unit 90 is arranged to generate the first axial depth profile 100-1 of the sample 50 based on the sampled first time-varying interference signal L_{I1}, and the second axial depth profile 100-2 of the sample 50 based on the sampled second time-varying interference signal L_{I2}, using well-known OCT data processing techniques. The first axial depth profile 100-1 may thus be a sideband image (or a harmonic image) of the sample 50, while the second axial depth profile 100-2 may be a real image of the sample 50.

The first axial depth profile 100-1 of the sample 50 generated by the data processing unit 90 may have complex conjugate mirror artifacts that are caused by the symmetry properties of the Fourier transform of a real-valued spectrum. These artifacts may overlap a portion of the desired image of the sample 50 in the first axial depth profile 100-1, making it difficult to distinguish real structures from the artifacts and thus rendering clinical information in the overlapping portion unreliable.

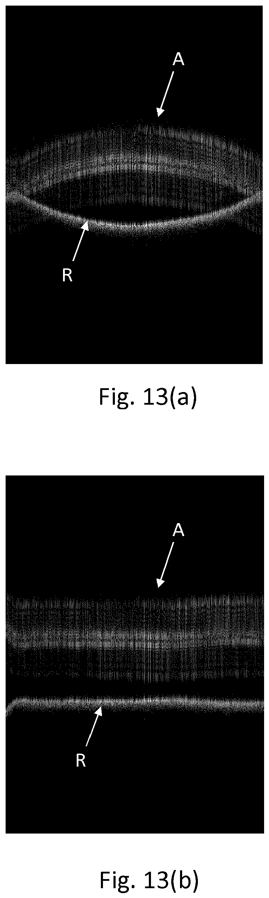

This problem is illustrated by the B-scan shown in Figure 13(a) where the real cross-sectional image R of a part of a 12 mm diameter plastic cylinder coated with electrical tape can be seen to be overlapped by a complex conjugate mirror artifact A. As shown in Figure 13(a), approximately half of the real image R overlaps to some extent with the artifact A. In a clinical application where the retina of an eye, for example, is imaged instead of the plastic cylinder, a substantial proportion of the imaged retina may likewise be corrupted by complex conjugate mirror artifacts, making the extraction of anatomical information from the affected region of the B-scan difficult or impossible.

Although complex conjugate mirror terms in individual A-scans can be effectively cancelled using more complex measurement set-ups or by employing post-processing techniques, such as dispersion-encoded full-range OCT, to produce full range tomograms, these tend to require additional hardware and/or are computationally expensive.

To address this problem, the OCT instrument 1 (or any of its variants of described herein) may, as in the present example embodiment, have the following configuration for acquiring, as the first axial depth profile 100-1 of the sample 50, an enhanced B-scan as described below, which represents a section of the sample 50, where the sample 50 in the section is inclined with respect to a plane normal to an axial direction along which depth information of the B-scan is acquired. For example, where the sample 50 comprises the retina of an eye, as in the present example embodiment, adjoining sections of the retina may have gradually varying inclinations relative to a plane normal to the axial direction such that the acquired image of the retina in the B-scan has a curvature. As another example, where the sample 50 comprises the anterior segment of an eye and a full-chamber anterior segment OCT (AS-OCT) image is acquired as the as the first axial depth profile 100-1, a line joining the scleral spurs at opposing sides of the section imaged in the AS-OCT image may be inclined relative to the plane. It is this curvature/inclination and the opposite curvature/inclination of the complex conjugate image that causes the image overlap and consequent problems described above. More particularly, the front-end optical system 40 may, as in the present example embodiment, be arranged to scan the signal light Lₛ₁ across a plurality of scan locations along the section of the sample 50, and the detector unit 60 may, as in this example embodiment, be arranged to sample, for each of the scan locations, a respective time-varying interference signal resulting from an interference between the first sideband light and the returned signal light Lₛ₂ (or the returned reference light Lᵣ₂, in variants where the adjustable optical frequency shifter 70 is provided in the sample arm of the interferometer). Furthermore, the data processing unit 90 may be arranged to generate the B-scan based on the sampled time-varying interference signals.

Moreover, the frequency shift controller 80-1 may in this case be further arranged to control the adjustable optical frequency shifter 70 to vary the optical frequency during the scan to compensate for the inclination of the sample 50 in the section, such that an image (or representation/depiction) of the sample 50 in the B-scan has less of an inclination relative to a lateral direction in the B-scan (or lateral axis in the B-scan, which is perpendicular to the axial (z) direction in the B-scan) than would be present in a B-scan of the same section of the sample 50 acquired by the OCT instrument 1 but without the control by the frequency shift controller 80-1 described herein. The frequency shift controller 80-1 may be configured to perform this function in one of several different ways.

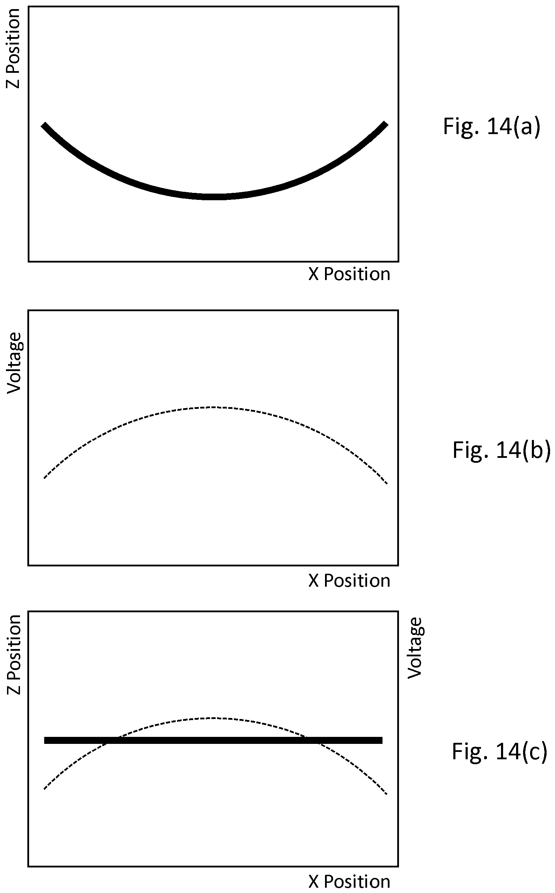

For example, the frequency shift controller 80-1 may, as in the present example embodiment, be arranged to control the adjustable optical frequency shifter 70 to vary the optical frequency during the acquisition of the B-scan to compensate for the inclination of the sample 50 in the section using pre-stored calibration data indicative of one or more measured inclinations of the sample 50 in the section. The calibration data may, for example, be obtained by acquiring, without any optical frequency shift or a constant optical frequency shift being applied by the adjustable optical frequency shifter 70, a calibration B-scan of the sample 50, as illustrated schematically in Figure 14(a). The curvature of the sample 50 in the calibration B-scan is then quantified, by using edge detection or selecting points manually, for example, and a function is then fitted to the resulting data. The function may take any suitable form, such as a linear or a polynomial function, for example. The function need not be given by an analytic expression, and may be numerically defined based on the resulting data. Regardless of its form, the function is then inverted and scaled (as necessary) to generate a control function, which can be used by the frequency shift controller 80-1 to drive the adjustable optical frequency shifter 70 with a varying voltage or other kind of control signal during the acquisition of a subsequent B-scan (preferably taken along the same scan path on the sample 50 as the calibration B-scan), as illustrated schematically in Figure 14(b).

The varying control signal causes the applied optical frequency shift to vary during the B-scan acquisition such that respective axial positions (e.g. centres) of the imaging depth ranges of component A-scans track the curvature of the sample 50. As a result, the A-scans in the subsequent B-scan are axially offset relative to one another such that the sample 50 appears substantially flat in the subsequent B-scan, as illustrated schematically in Figure 14(c). The scaling factor used to generate the control function may be determined by trial and error, such that a sufficiently flattened profile is obtained in the subsequent B-scan.

The result of a practical application of this technique to the B-scan of Figure 13(a) is shown in Figure 13(b). In this example, the function fitted to the real image in Figure 13(a) is a parabola, which was scaled manually to achieve the image flattening shown in Figure 13(b).

As the varying optical frequency shift that is applied by the adjustable optical frequency shifter 70 during the B-scan acquisition flattens not only the real image of the sample 50 in the subsequent B-scan but also the complex conjugate image, as shown in Figure 13(b), overlap of the real and complex conjugate images can be avoided or at least reduced. The control performed by the frequency shift controller 80-1 as described above can thus make it possible to extract image information that is not affected by conjugate artifacts from more of the B-scan than without the control.

It should be noted that, instead of controlling the adjustable optical frequency shifter 70 to vary the optical frequency during the acquisition of the B-scan to compensate for the inclination of the sample 50 in the section using pre-stored calibration data as described above, the frequency shift controller 80-1 may alternatively be arranged to control the adjustable optical frequency shifter 70 to vary the optical frequency during the acquisition of the B-scan to compensate for the inclination of the sample 50 in the section using a pre-stored estimate of the one or more inclinations of the sample 50 in the section, e.g. an estimated curvature of the sample 50 in the section thereof which is to be imaged. The estimate may be based on known general characteristics of the sample 50 (obtained from measurements of similar other samples, for example), and need only be sufficiently accurate to allow the overlap between the real and conjugate images to be avoided or at least reduced.

### [Second Example embodiment]

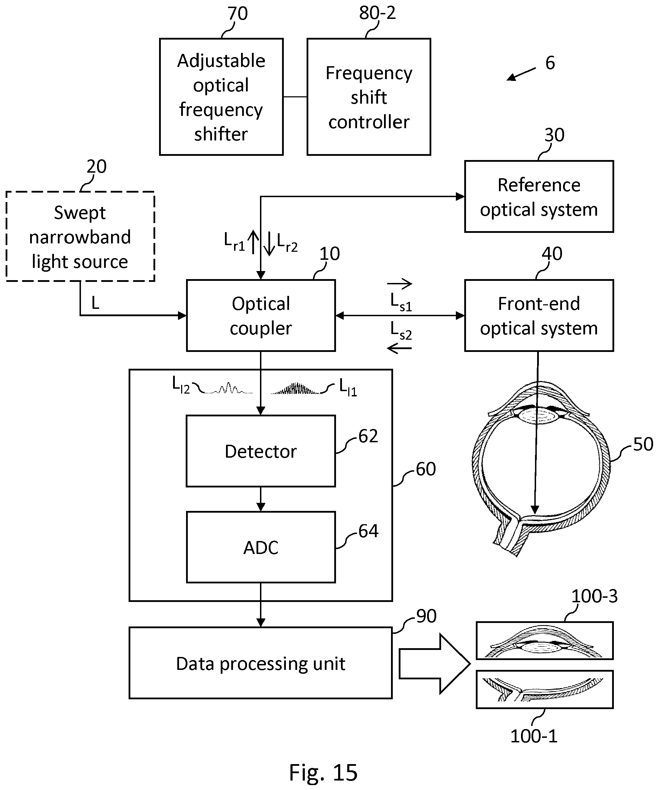

Figure 15 is a schematic diagram of an OCT instrument (also referred to herein as an OCT system) 6 according to a second example embodiment herein.

The present example embodiment differs from the first example embodiment described a above only by the configuration of the frequency shift controller 80-2, with all other components of the (swept-source) OCT instrument 6 and their arrangement within the OCT instrument 6 being the same as the like-numbered components of the OCT instrument 1 of the first example embodiment. In essence, the frequency shift controller 80-2 is operable to drive the adjustable optical frequency shifter 70 in a first mode and a second mode to generate respective sideband lights of difference frequencies, with interference light based the generated sideband lights being sampled and the samples being processed to generate sideband images (and no real image, as in the case of the first example embodiment) of the sample 50.

The frequency shift controller 80-2 is operable in a first mode to control the adjustable optical frequency shifter 70 to generate a first sideband light by adjustably increasing or decreasing an optical frequency of a part of the returned reference light Lᵣ₂, such that the detector unit 60 samples a first time-varying interference signal L_{I1} resulting from an interference between the first sideband light and the returned signal light Lₛ₂ as the optical frequency of the light L is being swept by the swept narrowband light source 20. In an alternative example embodiment (where the adjustable frequency shifter is provided in the sample arm), the frequency shift controller 80-2 may be operable in the first mode to control the adjustable optical frequency shifter 70 to generate the first sideband light by adjustably increasing or decreasing the optical frequency of some of the returned signal light Lₛ₂, such that the detector unit 60 samples a first time-varying interference signal L_{I1} resulting from an interference between the sideband light and the returned reference light Lᵣ₂ as the optical frequency of the light L is being swept by the swept narrowband light source 20. The first sideband light may, for example, correspond to the light of the positive first sideband generated by an electro-optic modulator (as an example of the adjustable frequency shifter 70).

The front-end optical system 40 may, as in the present example embodiment, be arranged to scan the signal light Lₛ₁ across the sample 50 in a first direction along a scan path on the sample 50 during control of the adjustable optical frequency shifter 70 in the first mode, such that the OCT instrument 6 acquires a first axial depth profile 100-1 in the form of a first B-scan showing a section of the sample 50 along the scan path.

The centre of the imaging depth range of the OCT instrument 6 in the first mode may be rapidly adjusted in the axial direction of the OCT instrument 6 by controlling the adjustable optical frequency shifter 70 to apply an appropriate optical frequency shift. Where the sample 50 is an eye of a subject, as in the present example embodiment, the centre of the imaging depth range of the OCT instrument 6 in the first mode may be set to coincide with the retina of the eye such that the first axial depth profile 100-1 (B-scan) shows a section of the retina, as shown in Figure 15.

The frequency shift controller 80-2 may, as in the present example embodiment, be further operable in a second mode to generate a second sideband light of different optical frequency than the first sideband light, by adjustably increasing or decreasing an optical frequency of a part of the returned reference light Lᵣ₂ such that the detector unit 60 samples a second time-varying interference signal L_{I2} resulting from an interference between the second sideband light and the returned signal light Lₛ₂. In the alternative example embodiment mentioned above, the frequency shift controller 80-2 may be operable in the second mode to control the adjustable optical frequency shifter 70 to generate the second sideband light by adjustably increasing or decreasing the optical frequency of some of the returned signal light Lₛ₂, such that the detector unit 60 samples a second time-varying interference signal L_{I2} resulting from an interference between the sideband light and the returned reference light Lᵣ₂. The second sideband light may, by way of example and without limitation, correspond to the light of the negative first sideband or positive second sideband generated by the adjustable frequency shifter 70.

During control of the adjustable optical frequency shifter 70 in the second mode, which follows its control by the frequency shift controller 80-2 in the first mode, the front-end optical system 40 may, as in the present example embodiment, be arranged to scan the signal light Lₛ₁ across the sample 50 along the scan path mentioned above but in a second direction opposite to the first direction, such that the OCT instrument 6 acquires a second axial depth profile 100-3 in the form of a second B-scan showing a section of the sample 50 along the scan path.

The centre of the imaging depth range of the OCT instrument 6 in the second mode may be rapidly adjusted in the axial direction of the OCT instrument 6 by controlling the adjustable optical frequency shifter 70 to apply an appropriate optical frequency shift. Where the sample 50 is an eye of a subject, as in the present example embodiment, the centre of the imaging depth range of the OCT instrument 6 in the second mode may be set to coincide with a point (e.g. a centre of the pupil) in the anterior segment of the eye, such that the second axial depth profile 100-3 (B-scan) shows a section of the anterior segment, as shown in Figure 15.

The OCT instrument 6 may thus be used to acquire a B-scan of the anterior segment of an eye and a B-scan of the retina of the eye through a common section of the eye, in rapid succession, by operation of the frequency shift controller 80-2 in the second mode while the OCT instrument 6 performs a forward scan to acquire an axial depth profile 100-3 in the form of a B-scan of the anterior segment of the eye, and by subsequent operation of the frequency shift controller 80-2 in the first mode while the OCT instrument 6 performs a reverse (back) scan along the same scan path to acquire a further axial depth profile 100-1, in the form of a B-scan of the retina of the eye. The order in which the anterior segment and retina of the eye are imaged may be reversed, such that the frequency shift controller 80-2 first operates in the first mode while the OCT instrument 6 performs a forward scan to acquire a B-scan of the retina of the eye, and by subsequent operation of the frequency shift controller 80-2 in the second mode while the OCT instrument 6 performs a reverse (back) scan along the same scan path to acquire a B-scan of the anterior segment of the eye. Furthermore, the anterior segment of the eye may alternatively be imaged during operation of the frequency shift controller 80-2 in the first mode, and the retina of the eye may be imaged during operation of the frequency shift controller 80-2 in the second mode.

When operating in the first and/or second mode, the frequency shift controller 80-2 may be arranged to control the adjustable optical frequency shifter 70 to vary the optical frequency during a scan to compensate for an inclination of the sample 50 in the section being imaged, as in the first example embodiment. By way of example and without limitation, the frequency shift controller 80-2 may be arranged to control the adjustable optical frequency shifter 70 in the first mode to vary the optical frequency such that retina appears substantially flat and horizontal in the first B-scan, and/or to control the adjustable optical frequency shifter 70 in the second mode to vary the optical frequency such that anterior segment appears substantially flat and horizontal in the second B-scan ("horizontal" here meaning orthogonal to the axial direction in the B-scan).

The data processing unit 90 is arranged to generate the first axial depth profile 100-1 of the sample 50 based on the sampled first time-varying interference signal L_{I1}, and the second axial depth profile 100-3 of the sample 50 based on the sampled second time-varying interference signal L_{I2}, using well-known OCT data processing techniques. The first axial depth profile 100-1 of the sample 50 and the second axial depth profile 100-3 of the sample are both sideband images in the present example embodiment. As the respective centres of the imaging depth ranges of the OCT instrument 6 for the first and second modes may be moved along the axial direction of the OCT instrument 6 by control of the optical frequency shift provided by the adjustable optical frequency shifter 70, the reference optical system 30 of the present example embodiment may be provided in the form of a non-electromechanical (or solid-state) reference optical system, which has no electrically driven moving parts to vary the optical path length of the reference arm.

### [Modifications and Variations]

Many modifications and variations may be made to the example embodiments described above.

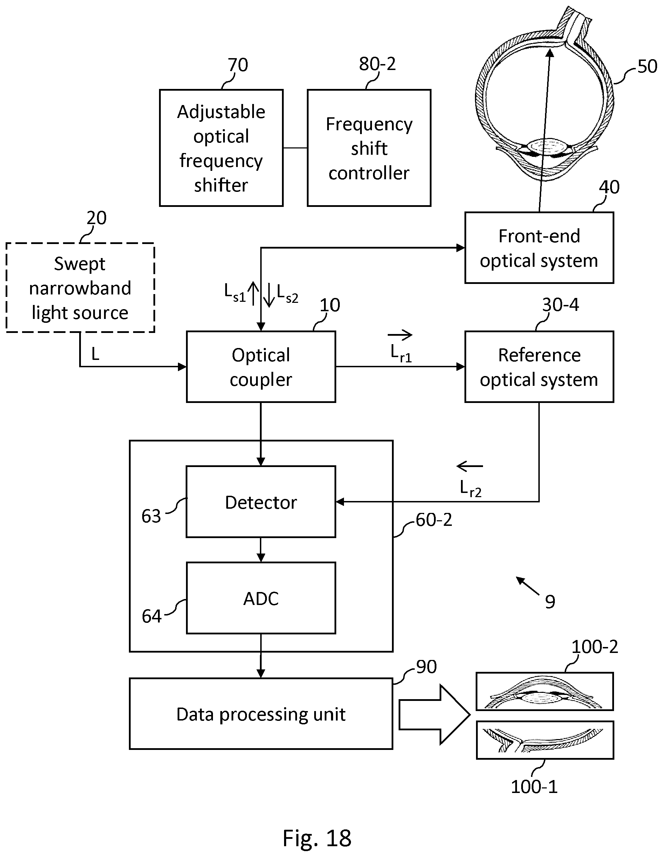

For example, although the detector unit 60 in each of the OCT instruments described above is arranged to sample the first time-varying interference signal L_{I1} and the second time-varying interference signal L_{I2} at different times, during operation of the frequency shift controller 80-1 or 80-2 in the first mode, e.g. while a forward B-scan of an imaging region is being acquired, followed by operation of the frequency shift controller 80-1 or 80-2 in the second mode, e.g. while a reverse B-scan of the imaging region is being acquired (or vice versa), an OCT instrument according to another example embodiment may be arranged such that the detector unit 60 thereof samples the first time-varying interference signal L_{I1} and the second time-varying interference signal L_{I2} concurrently. This may be achieved by using a balanced detector 63 as described below with reference to Figure 18, and splitting the electrical signal from the balanced detector 63 into two separate paths (i.e. generating two separate electrical signals corresponding to the result of the opto-electric conversion performed by the balanced detector 63), and applying appropriate filtering to each of these paths (electrical signals) as described above in relation to the first example embodiment, before passing the two resulting filtered signals to respective channels of a multichannel ADC connected to the balanced detector 63.

In an alternative example embodiment, concurrent sampling of the first time-varying interference signal L_{I1} and the second time-varying interference signal L_{I2} may be achieved by using a further optical coupler (e.g. an additional beam splitter) to split the return light propagating from the optical coupler 10 towards the detector unit 60 into a first interference light and a second interference light, and providing the detector unit 60 with a first detector and a second detector to detect the first interference light and the second interference light, respectively, using appropriate filtering of the electrical signal corresponding to the first interference light and/or the electrical signal corresponding to the second interference light, where necessary, to ensure that the f first time-varying interference signal L_{I1} and the second time-varying interference signal L_{I2} can be detected as described above.

As described above, the frequency shift controller 80-1 or 80-2 may be a radio frequency (which may include microwave frequency) driver arranged to apply a variable radio frequency (RF) or microwave frequency electric signal to the optical medium of the adjustable optical frequency shifter 70 using a suitable pair of capacitor plates attached to or arranged adjacent the optical medium, thereby to apply the variable electric field across the optical medium. Although lithium niobite (LiNbOs) is used as the medium for the electro-optic modulator in the example embodiment, other media may alternatively be used, including nonlinear or birefringent media. Example alternative media include potassium di-deuterium phosphate (KD*P or DKDP), potassium titanyl phosphate (KTP), beta-barium borate (BBO), lithium tantalate (LiTaO₃) and ammonium dihydrogen phosphate (NH₄H₂PO₄ or ADP). In addition to these inorganic media, nonlinear polymer media such as poled polymers may be used as the medium.

It should be noted that other nonlinear optical techniques may alternatively be used by the adjustable optical frequency shifter 70 to produce the sideband light via an appropriate frequency upshift or downshift. For example, difference-frequency generation or half-harmonic generation using, for example, a nonlinear optical crystal may be used instead of the acousto-optic modulator or electro-optic modulator to introduce a desired frequency shift.

Although the example embodiments described above employ free-space optics, those arrangements are not limiting to the scope of the invention. Indeed, in other example embodiments herein, optical fibre-based configurations can be employed, in which optical fibres are used in place of free space to propagate beams of light.

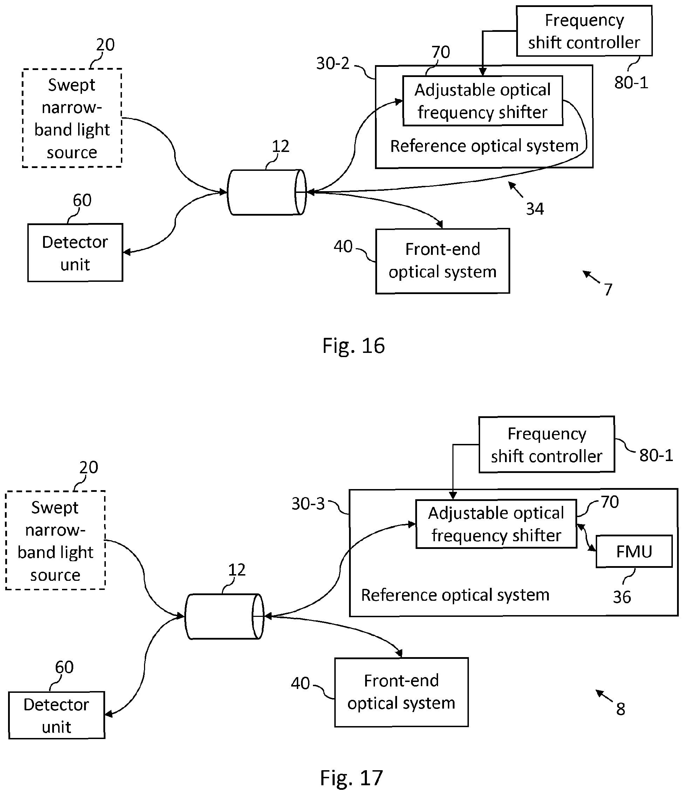

For example, Figure 16 is a schematic illustration of components of an OCT instrument 7 (also referred to herein as an "OCT system") according to another example embodiment herein. In Figure 16, light from swept narrowband light source 20 travels via an optical fibre to a fibre coupler unit (FCU) 12, which is an example of the optical coupler 10. The light from the swept narrowband light source 20 thereby splits into a reference light and a signal sample. The reference light is forwarded to a reference optical system 30-2 in a reference arm, circulates via adjustable optical frequency shifter 70 controlled by frequency shift controller 80-1, and returns to FCU 12. The signal sample is forwarded in a sample arm and travels via front-end optical system 40 to the sample (not shown in Figure 16), which reflects the signal sample so that it returns to FCU 12 by way of front-end optical system 40. At FCU 12, the returning light received from each arm is combined and passed along a further optical fibre to detector unit 60. Otherwise, the example embodiment of Figure 16 operates in the same way as the that of Figure 3, except that, since the light in the reference arm passes through the adjustable optical frequency shifter 70 only once, only a single, rather than a double, modulation is applied. The OCT instrument 6 of the second example embodiment described above with reference to Figure 15 may similarly be implemented in such an optical fibre-based configuration.

Components of an OCT instrument 8 according to a further example embodiment are illustrated in Figure 17. The OCT instrument 8 of Figure 17 includes the same components as those of Figure 16, except that reference optical system 30-3 of the embodiment of Figure 17 differs from reference optical system 30-2 of the embodiment of Figure 16. In Figure 17, the reference beam traveling in the reference arm is provided, by way of the adjustable optical frequency shifter 70, to a fibre mirror unit (FMU) 36. The FMU 36 reflects incident light received from the adjustable optical frequency shifter 70 and returns reflected light via the adjustable optical frequency shifter 70 to the FCU 12. Thereby, a double modulation is introduced in the same manner as in the free-space optical arrangement of Figure 5. The OCT instrument 6 of the second example embodiment described above with reference to Figure 15 may similarly be implemented in such an optical fibre-based configuration.

Furthermore, the example embodiments of Figures 1, 5 and 9-11 and 15-17 are implemented using so-called unbalanced detection, in which the interfering frequency components are directly detected from an optical signal by detector 62. However, in other example embodiments herein, a balanced detection arrangement can be employed instead, in which the return lights from the signal and reference arms are combined at a (e.g. 50:50) coupler and the outputs of the coupler are coupled to different optical inputs of a balanced detector. In such an arrangement, the intensity of the signal and reference light may also independently be measured, for example, by diverting at least some of each of the sample and reference light to a separate detection element, such that variations in the intensity of light can be compensated by the detection elements.

Figure 18 illustrates an OCT instrument 9 according to another example embodiment herein, which employs balanced detection. The OCT instrument 9 of Figure 18 is similar to the OCT instrument 1 of Figure 1, except that, in the OCT instrument 9 of Figure 18, the detector 63 comprises two optical inputs, and the reference optical system 30-4 is included in a loop optical system between an output of optical coupler 10 and one of the two optical inputs of detector 63. The loop optical system comprises the optical coupler 10, the reference optical system 30-4, and the detector 63. The optical loop may have a fixed optical path length, although this example is not limiting. Interference between the returning reference and signal sample provides the interferogram. The OCT instrument 6 of the second example embodiment described above with reference to Figure 15 may be similarly adapted to employ balanced detection in this way.

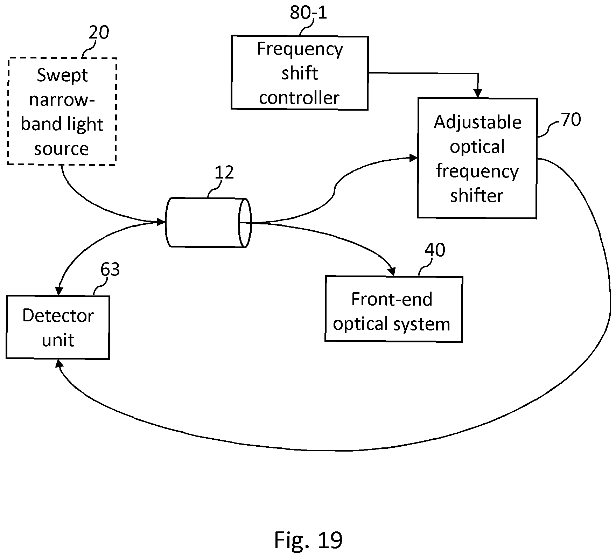

Figure 19 illustrates an OCT instrument according to another example embodiment herein, which has an optical fibre configuration. The OCT instrument of Figure 19 includes similar components as OCT instrument 7 of Figure 16, except that the reference optical system 30-2 is not represented in Figure 19 and, instead of an output of the adjustable optical frequency shifter 70 being provided to the FCU 12 as represented in Figure 17, in the OCT instrument of Figure 19, the output of the adjustable optical frequency shifter 70 is provided to a first one of plural inputs of detector 63. Accordingly, an optical fibre loop is provided that includes the FCU 12, the adjustable optical frequency shifter 70, and the detector 63. As such, a reference beam output by the FCU 12 is provided to adjustable optical frequency shifter 70, and an output of adjustable optical frequency shifter 70 is provided to the first input of the detector 63. A second input of detector 63 receives returning signal sample which returns from front-end optical system 40 through FCU 12. The interference between the returning reference and signal sample in the detector 63 provides the interferogram. The OCT instrument of Figure 19 may be modified to include frequency shift controller 80-2 of the second example embodiment instead of frequency shift controller 80-1.

The above configurations have been described in relation to discrete control and data processing units, such as the internal controller of swept narrowband light source 20, the frequency shift controller 80-1 or 80-2 controlling adjustable optical frequency shifter 70 and the data processing unit 90 transforming the detected optical signal of detector unit 60 into axial depth profiles 100-1 and 100-2 using a Fourier transform. However, in other example embodiments herein, at least some or all of the control and data processing aspects of the above configurations may be provided by an integrated controller 200 which may be instantiated as a programmable logic unit (PLU), application-specific integrated circuit (ASIC), a supervisory control and data acquisition system (SCADA), a general purpose data processor such as a microcomputer, minicomputer, or personal computer (PC), or a mobile device such as a tablet computer or smartphone.

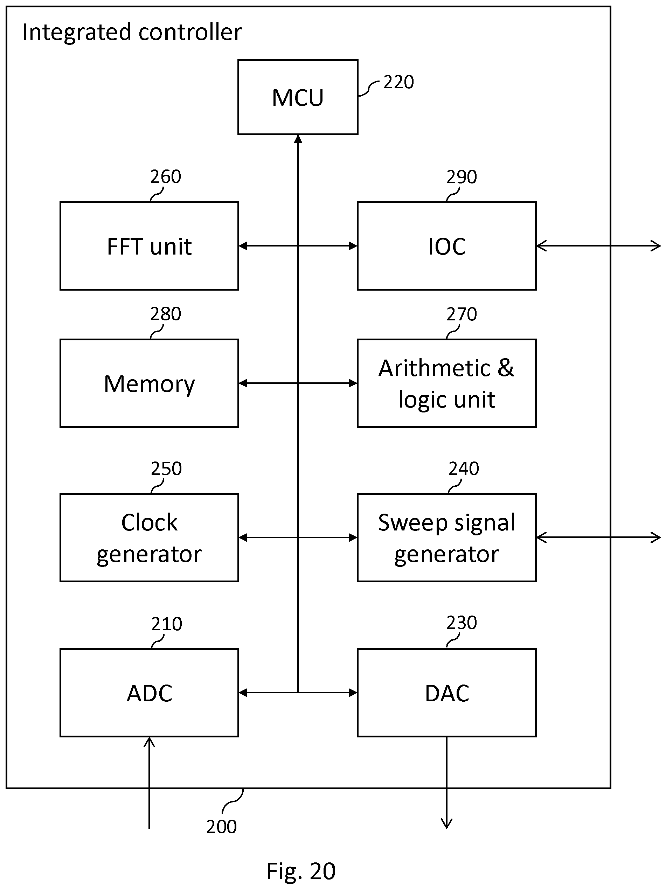

A schematic diagram of an example integrated controller 200 is shown in Figure 20. In one example embodiment herein, the integrated controller 200 controls at least some or all of the various components shown in Figures 1, 3, 5, and 9-11 and 15-19, and also may form all or at least part of the frequency shift controller 80-1 or 80-2. As represented in Figure 20, all of the components of the integrated controller 200 are coupled to one another and thus can inter-communicate. Integrated controller 200 comprises an ADC 210 to receive, quantize and sample signals from a detector (not shown in Figure 20), and, in one example embodiment herein, provide resulting converted signals to one or more other components of integrated controller 200, such as, by example only, a master control unit (MCU) 220. In one example embodiment herein, integrated controller 200 further comprises a digital to analogue converter (DAC) 230 that converts digital signals received from one or more other components of the integrated controller 200, such as, by example only, the MCU 220, to analogue value(s) and provides them as analogue control voltage(s) to a frequency shift controller (not shown in Figure 20), to thereby define an amount of frequency shift introduced by the adjustable optical frequency shifter 70 (not shown in Figure 20). Integrated controller 200 further comprises a sweep signal generator 240, which outputs a time-varying signal for control of frequency of the swept narrowband light source 20 (not shown in Figure 20. For example, the output of sweep signal generator 240 may be a time-varying voltage, and the swept narrowband light source 20 may accept the voltage input to define the centre frequency of the output narrowband light L. Integrated controller 200 also comprises a clock generator 250 which defines one or more clocks for providing a common time base for the operation of time-varying aspects of the integrated controller 200. For example, clock generator 250 may define a sample clock for ADC 210 as well as a time base for sweep signal generator 240. Clock generator 250 may also define internal clocks of integrated controller 200 such as memory and instruction clocks and data bus clocks. Integrated controller 200 further comprises a fast Fourier transform (FFT) unit 260 to perform Fourier transform on quantised and sample signals obtained via ADC 210. Integrated controller 200 also comprises an arithmetic and logic unit 270 for performing arithmetical logical operations on data handled within integrated controller 200.

Integrated controller 200 also comprises a memory 280, which in one example embodiment herein is computer-readable, for storing and retrieving data values such as recorded data from ADC 210, signal waveforms associated with sweep signal generator 240, Fourier-transformed output data generated by FFT unit 260, and other parameters, instructions and values as necessary for performing the operations of integrated controller 200. The memory 280 may comprise, by example only and without limitation, a RAM, ROM, hard drive, floppy disc, memory stick, a buffer, or the like. In one example embodiment herein, the memory 280 stores instructions and/or programs for performing the functions described herein and represented in the drawings. Integrated controller 200 also comprises an input/output (I/O) controller 290 for sending and receiving values to external devices such as off-line storage instantiated as a hard drive, flash drive or disk drive, or an interface such as a network interface, for example a wired local area network, a wireless area network, or a mobile data network. The master control unit 220 coordinates operations of the various functional units of integrated controller 200, and thus controls the other components of integrated controller 200. In one example embodiment herein, the master control unit 220 (and/or the arithmetic and logic unit 270) can read and write data, instructions, and programs from/to the memory 280, and can execute the instructions and programs to perform the functions described herein and represented in the drawings. Also in an example embodiment herein, the ADC 210 may form and/or be included in the ADC 64 of the other figures described herein, and the master control unit 220 and/or the integrated controller 200 may form and/or be included in the frequency shift controller 80-1 (or 80-2) and/or the data processing unit 90 of the other figures described herein. However, the division of various functional tasks into units as shown in Figure 20 is purely exemplary, and such tasks may be performed by individual functional modules, discrete electronics, integrated logic, other hardware, software and/or other program code, as required.

The disclosed apparatus may be implemented in a scanning laser ophthalmoscope (SLO), according to one example embodiment herein. Alternatively, the disclosed apparatus may be implemented to measure tissue other than the retina (e.g. at least a portion of the anterior chamber, or any other part of the eye), and other than tissue of the eye. For example, the disclosed apparatus and methods may be implemented to measure other biological membranes such as skin or plant parts, or may be applied to measure non-biological structures.

It should be noted that, although for convenience the ADC 64 and data processing unit 90 are not represented in Figures 5 and 9-11, 16, 17 and 19, the OCT instruments (systems) of those figures also include those components, with the data processing unit 90 being coupled to the detector unit 60 (or 63) of those respective OCT instruments.

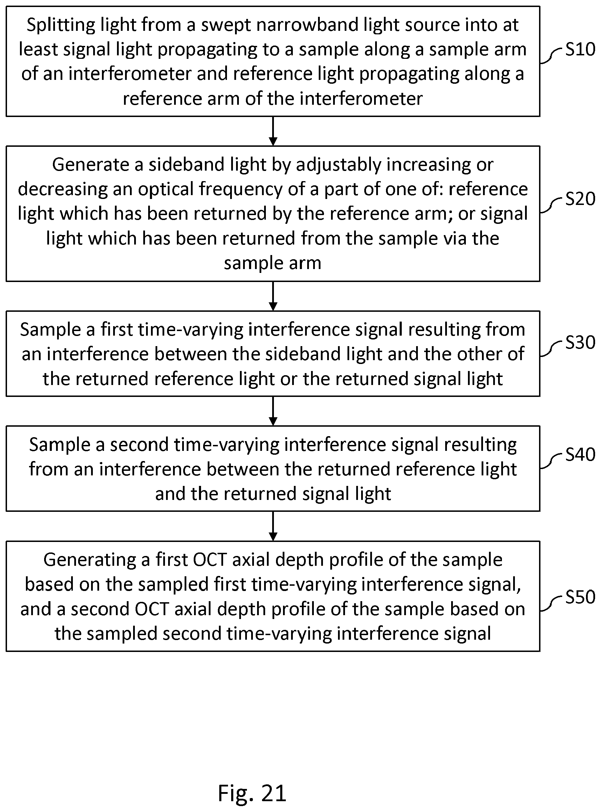

A method of generating OCT axial depth profiles of a sample 50 in accordance with the first example embodiment will now be described with reference to Figure 21.

In process S10 of Figure 21, light L from a swept narrowband light source 20 is split into at least signal light Lₛ₁ propagating to the sample 50 along a sample arm of an interferometer and reference light Lᵣ₁ propagating along a reference arm of the interferometer.

In process S20 of Figure 21, a sideband light is generated by adjustably increasing or decreasing an optical frequency of a part of one of: reference light Lᵣ₂ which has been returned by the reference arm; or signal light Lₛ₂ which has been returned from the sample 50 via the sample arm.

In process S30 of Figure 21, a first time-varying interference signal L_{I1}, resulting from an interference between the sideband light and the other of the returned reference light Lᵣ₂ or the returned signal light Lₛ₂, is sampled.

In process S40 of Figure 21, a second time-varying interference signal L_{I2}, resulting from an interference between the returned reference light Lᵣ₂ and the returned signal light Lₛ₂, is sampled.

In process S50 of Figure 21, a first OCT axial depth profile 100-1 of the sample 50 is generated based on the sampled first time-varying interference signal L_{I1}, and a second OCT axial depth profile 100-2 of the sample 50 is generated based on the sampled second time-varying interference signal L_{I2}.

It should be noted that the order in which some of processes described above are performed need not be as shown in Figure 21, and some of the processes may be performed in reverse order or concurrently. For example, process S40 may be performed before process S30, or these processes may be performed concurrently.

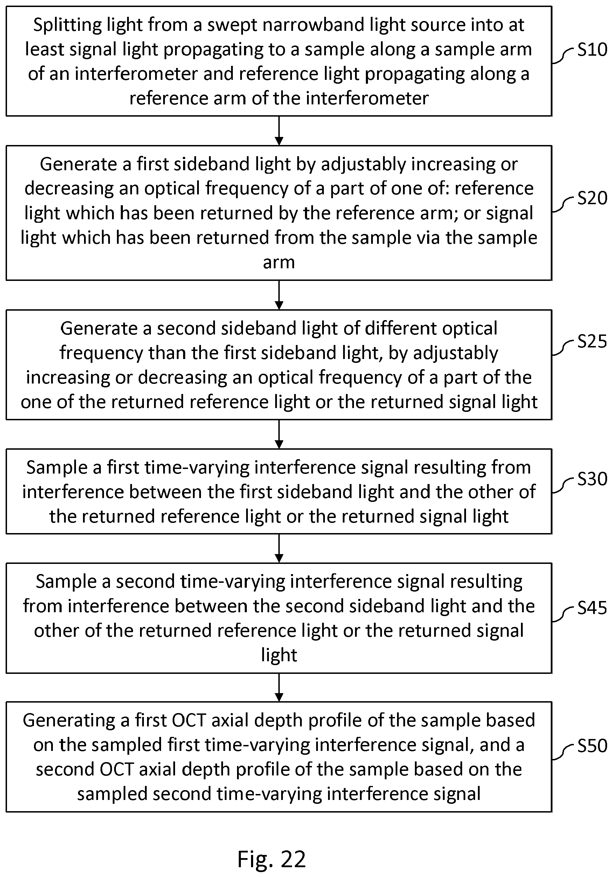

A method of generating OCT axial depth profiles of a sample 50 in accordance with the second example embodiment will now be described with reference to Figure 22.

In process S10 of Figure 22, light L from a swept narrowband light source 20 is split into at least signal light Lₛ₁ propagating to the sample 50 along a sample arm of an interferometer and reference light Lᵣ₁ propagating along a reference arm of the interferometer.

In process S20 of Figure 22, a sideband light is generated by adjustably increasing or decreasing an optical frequency of a part of one of: reference light Lᵣ₂ which has been returned by the reference arm; or signal light Lₛ₂ which has been returned from the sample 50 via the sample arm.

In process S25 of Figure 22, a second sideband light of different optical frequency than the first sideband light is generated by adjustably increasing or decreasing an optical frequency of a part of the one of the returned reference light Lᵣ₂ or the returned signal light Lₛ₂.

In process S30 of Figure 22, a first time-varying interference signal L_{I1}, resulting from an interference between the first sideband light and the other of the returned reference light Lᵣ₂ or the returned signal light Lₛ₂, is sampled.

In process S45 of Figure 22, a second time-varying interference signal L_{I2}, resulting from an interference between the second sideband light and the other of the returned reference light Lᵣ₂ or the returned signal light Lₛ₂, is sampled.

In process S50 of Figure 22, a first OCT axial depth profile 100-1 of the sample 50 is generated based on the sampled first time-varying interference signal L_{I1}, and a second OCT axial depth profile 100-2 of the sample 50 is generated based on the sampled second time-varying interference signal L_{I2}.

It should be noted that the order in which some of processes described above with reference to Figure 22 are performed need not be as shown in Figure 22, and some of the processes may be performed in reverse order or concurrently. For example, process S25 may be performed before process S20, or these processes may be performed concurrently. Where processes S20 and S25 are performed concurrently, process S45 may be performed before or after process S30, or processes S30 and S45 may be performed concurrently. Furthermore, the method may comprise performing process S10, followed by process S20, followed by process S30, followed by process S25, followed by process S45, followed by process S50.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the OCT instruments or electronic components thereof described herein may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

Further, the purpose of the Abstract is to enable the Patent Office and the public generally, and especially the scientists, engineers and practitioners in the art who are not familiar with patent or legal terms or phraseology, to determine quickly from a cursory inspection the nature and essence of the technical disclosure of the application. The Abstract is not intended to be limiting as to the scope of the example embodiments presented herein in any way. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An optical coherence tomography instrument comprising:
an optical coupler (10) arranged to accept light (L) from a swept narrowband light source (20) and to split the light (L) into at least signal light (Lₛ₁) and reference light (Lᵣ₁);
a reference optical system (30) arranged to return the reference light (Lᵣ₁);
a front-end optical system (40) arranged to direct the signal light (Lₛ₁) towards a sample (50) and to return signal light (Lₛ₂) reflected from the sample (50);
a detector unit (60) arranged to sample at least one time-varying interference signal based on the returned reference light (Lᵣ₂) and the returned signal light (Lₛ₂);
an adjustable optical frequency shifter (70);
a frequency shift controller (80-1), which is arranged to control the adjustable optical frequency shifter (70) to:
generate a sideband light by adjustably increasing or decreasing an optical frequency of a part of one of the returned reference light (Lᵣ₂) or the returned signal light (Lₛ₂), such that the detector unit (60) samples a first time-varying interference signal (L_{I1}) resulting from an interference between the sideband light and the other of the returned reference light (Lᵣ₂) or the returned signal light (Lₛ₂); and
maintain the optical frequency of the one of the returned reference light (Lᵣ₂) or the returned signal light (Lₛ₂), such that the detector unit (60) samples a second time-varying interference signal (L_{I2}) resulting from an interference between the returned reference light (Lᵣ₂) and the returned signal light (Lₛ₂); and
a data processing unit (90) arranged to generate:
a first axial depth profile (100-1) of the sample (50) based on the sampled first time-varying interference signal (L_{I1}); and
a second axial depth profile (100-2) of the sample (50) based on the sampled second time-varying interference signal (L_{I2}).

2. The optical coherence tomography instrument according to claim 1, wherein
the frequency shift controller (80-1) is operable in a first mode to control the adjustable optical frequency shifter (70) to generate the sideband light, and in a second mode to control the adjustable optical frequency shifter (70) to maintain the optical frequency of the one of the returned reference light (Lᵣ₂) or the returned signal light (Lₛ₂), and
the front-end optical system (40) is arranged to scan the signal light (Lₛ₁) across the sample (50) in a first direction along a scan path on the sample (50) during operation of the frequency shift controller (80-1) in the first mode, and along the scan path in a second direction opposite to the first direction during operation of the frequency shift controller (80-1) in the second mode.

3. An optical coherence tomography instrument comprising:
an optical coupler (10) arranged to accept light (L) from a swept narrowband light source (20) and to split the light (L) into at least signal light (Lₛ₁) and reference light (Lᵣ₁);
a reference optical system (30) arranged to return the reference light (Lᵣ₁);
a front-end optical system (40) arranged to direct the signal light (Lₛ₁) towards a sample (50) and to return signal light (Lₛ₂) reflected from the sample (50);
a detector unit (60) arranged to sample at least one time-varying interference signal based on the returned reference light (Lᵣ₂) and the returned signal light (Lₛ₂);
an adjustable optical frequency shifter (70);
a frequency shift controller (80-2), which is arranged to control the adjustable optical frequency shifter (70) to:
generate a first sideband light by adjustably increasing or decreasing an optical frequency of a part of one of the returned reference light (Lᵣ₂) or the returned signal light (Lₛ₂), such that the detector unit (60) samples a first time-varying interference signal (L_{I1}) resulting from an interference between the first sideband light and the other of the returned reference light (Lᵣ₂) or the returned signal light (Lₛ₂); and
generate a second sideband light of different optical frequency than the first sideband light, by adjustably increasing or decreasing an optical frequency of a part of the one of the returned reference light (Lᵣ₂) or the returned signal light (Lₛ₂), such that the detector unit (60) samples a second time-varying interference signal (L_{I2}) resulting from an interference between the second sideband light and the other of the returned reference light (Lᵣ₂) or the returned signal light (Lₛ₂); and
a data processing unit (90) arranged to generate:
a first axial depth profile (100-1) of the sample (50) based on the sampled first time-varying interference signal (L_{I1}); and
a second axial depth profile (100-3) of the sample (50) based on the sampled second time-varying interference signal (L_{I2}).

4. The optical coherence tomography instrument according to claim 3, wherein
the frequency shift controller (80-2) is operable in a first mode to control the adjustable optical frequency shifter (70) to generate the first sideband light, and in a second mode to control the adjustable optical frequency shifter (70) to generate the second sideband light, and
the front-end optical system (40) is arranged to scan the signal light (Lₛ₁) across the sample (50) in a first direction along a scan path on the sample (50) during operation of the frequency shift controller (80-2) in the first mode, and along the scan path in a second direction opposite to the first direction during operation of the frequency shift controller (80-2) in the second mode.

5. The optical coherence tomography instrument according to claim 3 or claim 4, wherein the reference optical system (30) is non-electromechanical.

6. The optical coherence tomography instrument according to any preceding claim, wherein the sample (50) comprises an eye of a subject, and the first axial depth profile comprises an axial depth profile of a portion of a retina of the eye, and the second axial depth profile comprises an axial depth profile of a portion of an anterior segment of the eye.

7. The optical coherence tomography instrument according to any of claims 1 to 5, wherein
the optical coherence tomography instrument is arranged to acquire, as the first axial depth profile (100-1) of the sample (50), a B-scan representing a section of the sample (50), the sample (50) in the section being inclined with respect to a plane normal to an axial direction along which depth information of the B-scan is acquired,
the front-end optical system (40) is arranged to scan the signal light (Lₛ₁) across a plurality of scan locations along the section of the sample (50),
the detector unit (60) is arranged to sample, for each of the scan locations, a respective time-varying interference signal resulting from an interference between the sideband light and the other of the returned reference light (Lᵣ₂) or the returned signal light (Lₛ₂),
the data processing unit (90) is arranged to generate the B-scan based on the sampled time-varying interference signals, and
the frequency shift controller (80-1; 80-2) is further arranged to control the adjustable optical frequency shifter (70) to vary the optical frequency during the scan to compensate for the inclination of the sample (50) in the section, such that an image of the sample in the B-scan has less of an inclination relative to a lateral direction in the B-scan than would be present in a B-scan of the section of the sample (50) acquired by the optical coherence tomography instrument without the control by the frequency shift controller (80-1; 80-2).

8. The optical coherence tomography instrument according to claim 7, wherein the frequency shift controller (80-1; 80-2) is arranged to control the adjustable optical frequency shifter (70) to vary the optical frequency during the acquisition of the B-scan to compensate for the inclination of the sample (50) in the section using one of:
a pre-stored estimate of the inclination of the sample (50) in the section; or
pre-stored calibration data indicative of a measured inclination of the sample (50) in the section.

9. The optical coherence tomography instrument according to claim 7 or claim 8, wherein the sample (50) comprises a retina of an eye of a subject.

10. The optical coherence tomography instrument according to any preceding claim, wherein the reference optical system (30) comprises one of
a reflector (32) arranged to reflect the reference light (Lᵣ₁) to return the reference light (Lᵣ₁), or
an optical loop (34) to return the reference light (Lᵣ₁), the optical loop (34) optionally having a fixed optical path length.

11. The optical coherence tomography instrument according to any preceding claim, wherein one of the reference light (Lᵣ₁) or the signal light (Lₛ₁) passes by way of the adjustable optical frequency shifter (70) in forward and reverse directions.

12. The optical coherence tomography instrument according to any preceding claim, wherein the optical frequency shifter (70) includes an acousto-optic modulator or an electro-optic modulator.

13. The optical coherence tomography instrument according to any preceding claim, further comprising the swept narrowband light source (20), wherein the swept narrowband light source (20) is arranged to emit the light (L) to the optical coupler (10) and periodically vary an optical frequency of the light (L) emitted thereby, the light (L) being narrowband light.

14. A method of generating OCT axial depth profiles of a sample (50), the method comprising:
splitting (S10) light (L) from a swept narrowband light source (20) into at least signal light (Lₛ₁) propagating to the sample (50) along a sample arm of an interferometer and reference light (Lᵣ₁) propagating along a reference arm of the interferometer;
generating (S20) a sideband light by adjustably increasing or decreasing an optical frequency of a part of one of:
reference light (Lᵣ₂) which has been returned by the reference arm; or
signal light (Lₛ₂) which has been returned from the sample (50) via the sample arm;
sampling (S30) a first time-varying interference signal (L_{I1}) resulting from an interference between the sideband light and the other of the returned reference light (Lᵣ₂) or the returned signal light (Lₛ₂);
sampling (S40) a second time-varying interference signal (L_{I2}) resulting from an interference between the returned reference light (Lᵣ₂) and the returned signal light (Lₛ₂); and
generating (S50) a first OCT axial depth profile (100-1) of the sample (50) based on the sampled first time-varying interference signal (L_{I1}), and a second OCT axial depth profile (100-2) of the sample (50) based on the sampled second time-varying interference signal (L_{I2}).

15. A method of generating OCT axial depth profiles of a sample (50), the method comprising:
splitting (S10) light (L) from a swept narrowband light source (20) into at least signal light (Lₛ₁) propagating to the sample (50) along a sample arm of an interferometer and reference light (Lᵣ₁) propagating along a reference arm of the interferometer;
generating (S20) a first sideband light by adjustably increasing or decreasing an optical frequency of a part of one of:
reference light (Lᵣ₂) which has been returned by the reference arm; or
signal light (Lₛ₂) which has been returned from the sample (50) via the sample arm;
generating (S25) a second sideband light of different optical frequency than the first sideband light, by adjustably increasing or decreasing an optical frequency of a part of the one of the returned reference light (Lᵣ₂) or the returned signal light (Lₛ₂);
sampling (S30) a first time-varying interference signal (L_{I1}) resulting from an interference between the first sideband light and the other of the returned reference light (Lᵣ₂) or the returned signal light (Lₛ₂);
sampling (S45) a second time-varying interference signal (L_{I2}) resulting from an interference between the second sideband light and the other of the returned reference light (Lᵣ₂) or the returned signal light (Lₛ₂); and
generating (S50) a first OCT axial depth profile (100-1) of the sample (50) based on the sampled first time-varying interference signal (L_{I1}), and a second OCT axial depth profile (100-2) of the sample (50) based on the sampled second time-varying interference signal (L_{I2}).
